(19) Europäisches Patentamt
European Patent Office
Office européen des brevets

(11) Numéro de publication: **0 255 415 B1**

(12) **FASCICULE DE BREVET EUROPEEN**

(45) Date de publication de fascicule du brevet: **14.04.93**

(51) Int. Cl.$^5$: **A61K 31/135**, A61K 31/34

(21) Numéro de dépôt: **87401462.4**

(22) Date de dépôt: **25.06.87**

# (54) Utilisation de phényléthanolamines pour la préparation de médicaments agissant sur les troubles gastro-intestinaux.

(30) Priorité: **27.06.86 IT 2092486**

(43) Date de publication de la demande:
**03.02.88 Bulletin 88/05**

(45) Mention de la délivrance du brevet:
**14.04.93 Bulletin 93/15**

(84) Etats contractants désignés:
**BE CH DE FR GB IT LI LU NL SE**

(56) Documents cités:
**EP-A- 0 007 204**
**EP-A- 0 007 205**
**EP-A- 0 023 385**
**EP-A- 0 164 700**

**EUROPEAN JOURNAL OF PHARMACOLOGY, vol. 100, nos. 3/4, 1984, pages 309-319, Elsevier Science Publishing Co., Amsterdam, NL; C. WILSON et al.: "The rat lipolytic beta-adrenoceptor: studies using novel beta-adrenoceptor agonists"**

**"Algemeine und Spezielle Pharmakologie und Toxikologie", page 74: "Pharmakologie des peripheren autonomen Nervensystems"**

(73) Titulaire: **ELF SANOFI**
**32-34, rue Marbeuf**
**F-75008 Paris(FR)**
(84) Etats contractants désignés:
**BE CH DE FR GB LI LU NL SE**

(73) Titulaire: **MIDY S.p.A.**
**Via Piranesi 38**
**I-20137 Milano(IT)**
(84) Etats contractants désignés:
**IT**

(72) Inventeur: **Manara, Luciano**
**Via Novella 2/2 Pietra Marazzi**
**I-Alessandria(IT)**
Inventeur: **Tiziano, Croci**
**Via Gracinti 2 Rozzano**
**I-Milan(IT)**
Inventeur: **Bianchetti, Alberto**
**Via Corridoni 11**
**I-Milan(IT)**

(74) Mandataire: **Gillard, Marie-Louise et al**
**Cabinet Beau de Loménie 158, rue de l'Université**
**F-75340 Paris Cédex 07 (FR)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).



Rank Xerox (UK) Business Services
(3.10/3.5x/3.0.1)

NATURE, vol. 309, no. 5964, 10 mai 1984, pages 163-165; J.R.S. ARCH et al.: "Atypical beta-adrenoceptor on brown adipocytes as target for anti-obesity drugs"

LIFE SCIENCE, vol. 32, 28 mars 1983, pages 1515-1522, Pergamon Press Ltd., US; T.T. YEN et al.: "Stimulation of cyclic AMP and lipolysis in adipose tissue of normal and obese Avy/a mice by LY79771, a phenethanolamine, and steroisomers"

INTERNATIONAL JOURNAL OF OBESITY, vol. 8, supplément 1, 1984, pages 65-78; T.T. YEN et al.: "Thermogenesis and weight control"

DIABETOLOGIA, vol. 27, no. 2, 1984, page 291A, résumé no. 242: D. ISLER et al.: "Anti-diabetic activity of Ro 16-8714, a beta-adrenergic agonist, in obese hyperglycaemic (ob/ob) mice and streptozotocia-diabetic rats"

FED. PROC., vol. 44, no. 4, 1985, page 1159, résumé 4377; S. HOGAN et al.: "Antiobesity activity of Ro 16-8714 in diet-induced obese (DIO) rats"

GOODMAN AND GILMAN'S: "The pharmacological basis of therapeutics", 7ième édition, 1985, pages 145-150, chapter 8, Macmillan Publishing Co., New York, US; N. WEINER: "Norepinephrine, epinephrine, and the sympathomimetic amines"

RECUEIL TRAV. CHIM. PAYS-BAS, vol. 92, 1973, pages 1281-1291; J. VAN DIJK et al.: "Synthesis of beta-phenylethylamine derivatives X1* N-(hydroxy- and methoxy-aralkyl) derivatives"

## Description

La présente invention concerne l'utilisation de certains analogues de phényléthanolamines et de leurs sels pharmaceutiquement acceptables pour la préparation de compositions pharmaceutiques destinées à la prophylaxie et/ou au traitement des troubles gastro-intestinaux associés à une contraction du muscle lisse sans effets cardiaques appréciables. La présente invention concerne également des compositions pharmaceutiques pour la même indication.

WILSON et al. dans EUROPEAN JOURNAL OF PHARMACOLOGY, 100 (1984) 309-319 font part de leurs études sur l'activité de certains composés $\beta$-agonistes classiques et d'autres composés $\beta$-agonistes nouveaux: les composés BRL, sur l'atrium, l'utérus et le tissu adipeux du rat. Ils ont mis en évidence que les composés BRL, à la différence des autres $\beta$-agonistes ($\beta_1$ et $\beta_2$ classiques) ont une sélectivité pour la lipolyse du tissu adipeux, activité qui justifie la proposition de les utiliser dans le traitement de l'obésité.

Le schéma (page 74) de l'ouvrage ALLGEMEINE und SPEZIELLE PHARMAKOLOGIE UND TOXIKOLOGIE indique seulement qu'aussi bien les récepteurs $\alpha$ que les récepteurs $\beta$ sont des médiateurs de la réponse contractile du muscle lisse du système gastro-intestinal

Les enseignements de ces deux documents ne donnent aucune indication sur l'existence ou non de composés ayant une activité spasmolytique sélective sur le tractus intestinal.

Les demandes de brevets européens publiées sous les n. 6735, 7204, 21636, 23385, 25331, 28105, 40000, 40915, 52963, 61907, 63004, 66351, 68669, 70133, 70134, 89154, 91749, 95827, 99707, 101069, 140359, 146392, 164700, 170121, 170135, 171519, le brevet belge 900.983, la demande de brevet GB 2133986, la demande de brevet australien publiée sous le n. 84/31944, la demande de brevet internationale publiée sous le n. WO 84/00956 et le brevet US 4.391.826 décrivent des phényléthanolamines, éventuellement modifiées pour former des éthers, des esters ou des dérivés cycliques, ayant une activité antihyperglycémiante et/ou antiobésité.

On a maintenant trouvé que les phényléthanolamines, leurs dérivés ou analogues décrits dans les brevets ci-dessus, ainsi que leurs sels, peuvent être utilisés pour la préparation de compositions pharmaceutiques actives sur le muscle lisse intestinal, destinées au traitement de troubles associés à une contraction du muscle lisse sans comporter d'effets secondaires cardiaques ou respiratoires importants, pourvu que lesdits produits ne contiennent pas de groupes hydroxy dans le noyau phényle de la phényléthanolamine.

Ainsi, la présente invention concerne, selon un de ses aspects, l'utilisation d'un analogue de phényléthanolamine ou d'un de ses sels pharmaceutiquement acceptables pour la préparation de compositions pharmaceutiques destinées à la prophylaxie et/ou au traitement des troubles gastro-intestinaux associés à une contraction du muscle lisse, ledit analogue de phényléthanolamine ayant la formule.

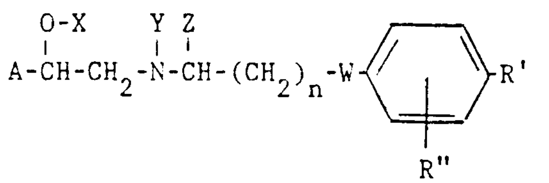

I

dans laquelle:

- n est 1, 2 ou 3;
- A représente un groupe benzofuran-2-yle ou un groupe phényle non substitué ou substitué par un ou deux atomes d'halogène ou par un groupe alkyle inférieur ou trifluorométhyle;
- R' représente
  . l'hydrogène;
  . un groupe ($C_1$-$C_6$) alkyle;
  . un groupe fonctionnel choisi parmi les groupements suivants: hydroxy; ($C_1$-$C_6$) alcoxy; ($C_2$-$C_6$) alcényloxy; ($C_2$-$C_6$) alcynyloxy; ($C_3$-$C_8$) cycloalkyloxy; ($C_3$-$C_8$) cycloalkyl-alcoxy; benzyloxy; phénoxy; mercapto; ($C_1$-$C_6$) alkyle thio; ($C_2$-$C_6$) alcényle thio; ($C_2$-$C_6$) alcynyle thio; ($C_3$-$C_8$) cycloalkylthio; ($C_3$-$C_8$) cycloalkyl-($C_1$-$C_6$) alkyle thio; benzylthio; phénylthio; (($C_1$-$C_6$) alkyle)sulfinyle; (($C_2$-$C_6$) alcényle)sulfinyle; (($C_2$-$C_6$) alcynyle)sulfinyle; ($C_3$-$C_8$) cycloalkylsulfinyle; (($C_3$-$C_8$) cycloalkyl-($C_1$-$C_6$) alkyle)sulfinyle; benzylsulfinyle; phénylsulfinyle; (($C_1$-$C_6$) alkyle)sulfonyle; (($C_2$-$C_6$) alcényle)-sulfonyle; (($C_2$-$C_6$) alcynyle)sulfonyle; ($C_3$-$C_8$) cycloalkylsulfonyle; (($C_3$-$C_8$) cycloalkyl-($C_1$-$C_6$) alkyle)sulfonyle; benzylsulfonyle; phénylsulfonyle; cyano; nitro; amino non substitué ou substitué par un

ou deux radicaux, identiques ou différents, choisis parmi ($C_1$-$C_6$) alkyle, ($C_2$-$C_6$) alcényle, ($C_2$-$C_6$) alcynyle, ($C_3$-$C_8$) cycloalkyle, ($C_3$-$C_8$) cycloalkyl-($C_1$-$C_6$) alkyle, benzyle, phényle; carboxy; carbalcoxy dont le groupe alkyle est en $C_1$-$C_6$; (($C_2$-$C_6$) alcényloxy)carbonyle; (($C_2$-$C_6$) alcynyloxy)-carbonyle; ($C_3$-$C_8$) cycloalkyloxycarbonyle; (($C_3$-$C_8$) cycloalkyl-($C_1$-$C_6$) alcoxy)carbonyle; benzyloxycarbonyle; phénoxycarbonyle; carbamoyle non substitué ou substitué sur le groupe amino par un ou deux radicaux, identiques ou différents, choisis parmi les groupes ($C_1$-$C_6$) alkyle, ($C_2$-$C_6$) alcényle, ($C_2$-$C_6$) alcynyle inférieur, ($C_3$-$C_8$) cycloalkyle, ($C_3$-$C_8$) cycloalkyl-($C_1$-$C_6$) alkyle, benzyle, phényle;

. un radical R choisi parmi les groupes ($C_1$-$C_6$) alkyle substitués par un groupe fonctionnel, les groupes ($C_2$-$C_6$) alcényle substitués par un groupe fonctionnel, les groupes ($C_2$-$C_6$) alcynyle substitués par un groupe fonctionnel, les groupes phényl(($C_1$-$C_6$) alkyle) substitués sur le groupe phényle par un ($C_1$-$C_6$) alkyle ou par un groupe fonctionnel, les groupes phényl(($C_2$-$C_6$) alcényle) substitués sur le groupe phényle par un ($C_1$-$C_6$) alkyle ou par un groupe fonctionnel, les groupes phényl(($C_2$-$C_6$) alcynyle) substitués sur le groupe phényle par un ($C_1$-$C_6$) alkyle ou par un groupe fonctionnel, les groupes benzyle substitués sur le groupe phényle par un ($C_1$-$C_6$) alkyle ou par un groupe fonctionnel, ou les groupes phényle non substitués ou substitués par un ($C_1$-$C_6$) alkyle ou par un groupe fonctionnel, le groupe fonctionnel étant tel que défini ci-dessus;

. un groupe O-R, S-R, SO-R ou $SO_2R$ dans lesquels R est tel que défini ci-dessus,

. un groupe NRR°, où R est tel que défini ci-dessus et R° représente l'hydrogène ou est tel que défini ci-dessus pour R, ou bien R et R° forment, avec l'atome d'azote auquel ils sont liés, un groupe choisi parmi les groupes pyrrolidino, pipéridino et morpholino;

. un groupe COOR ou un groupe CO-SR, dans lesquels R est tel que défini ci-dessus,

. un groupe CONRR°, où R est tel que défini ci-dessus et R° représente l'hydrogène ou est tel que défini ci-dessus pour R, ou bien R et R° forment, avec l'atome d'azote auquel ils sont liés, un groupe choisi parmi les groupes pyrrolidino, pipéridino et morpholino;

. un groupe $SO_2NRR°$, où R est tel que défini ci-dessus et R° représente l'hydrogène ou est tel que défini ci-dessus pour R, ou bien R et R° forment, avec l'azote auquel ils sont liés, un groupe choisi parmi les groupes pyrrolidino, pipéridino et morpholino;

- R'' représente
  . l'hydrogène;
  . un halogène;
  . un ($C_1$-$C_6$) alkyle;
  . un groupe fonctionnel tel que défini ci-dessus;
  . un groupe O-R, R étant tel que défini ci-dessus;
  . un groupe COOR, R étant tel que défini ci-dessus;
  . un groupe CONRR°, où R est tel que défini ci-dessus et R°, représente l'hydrogène ou est tel que défini ci-dessus pour R, ou bien R et R° forment, avec l'azote auquel ils sont liés, un groupe choisi parmi les groupes pyrrolidino, pipéridino et morpholino;
- W représente une liaison directe ou un atome d'oxygène;
- X représente l'hydrogène, un ($C_1$-$C_6$) alkyle ou un ($C_1$-$C_6$) alkylcarbonyle;
- Y représente l'hydrogène ou un groupe A' -CH(OH)-$CH_2$-, A' étant identique à A, mais autre que benzofuran-2-yle; ou bien
- X et Y, pris ensemble, forment un groupe méthylène, éventuellement substitué par un groupe carbalcoxy dont le groupe alkyle est en $C_1$-$C_6$,; un groupe éthylène, éventuellement substitué par un groupe oxo; ou un groupe 1,3-propylène; et
- Z représente l'hydrogène ou un ($C_1$-$C_6$) alkyle.

Les termes "($C_1$-$C_6$) alkyle", "($C_2$-$C_6$) alcényle" et "($C_2$-$C_6$) alcynyle", tels qu'utilisés ici, désignent les radicaux monovalents dérivés d'hydrocarbures aliphatiques saturés ou contenant une double ou une triple liaison et ayant jusqu'à 6 atomes de carbone, tel que par exemple les groupes méthyle, éthyle, propyle, isopropyle, t-butyle, néopentyle, allyle, crotyle, propargyle.

Le terme "($C_3$-$C_8$) cycloalkyle" désigne le radical monovalent d'un hydrocarbure alicyclique contenant de 3 à 8 atomes de carbone, le cyclopropyle, le cyclopentyle et le cyclohexyle étant particulièrement préférés.

Les termes "alcoxy dont le groupe alkyle est en $C_1$-$C_6$", "($C_2$-$C_6$) alcényloxy", "($C_2$-$C_6$) alcynyloxy", "-($C_3$-$C_8$) cycloalkyloxy" et les dérivés soufrés correspondants, tels qu'utilisés ici, désignent le groupe hydroxyle ou thiol éthérifié par un ($C_1$-$C_6$) alkyle, par un ($C_2$-$C_6$) alcényle, par un ($C_2$-$C_6$) alcynyle ou par un ($C_3$-$C_8$) cycloalkyle, tels que définis ci-dessus.

Le terme "carbalcoxy" désigne un ester de ($C_1$-$C_6$) alkyle du groupe carboxyle, le terme ($C_1$-$C_6$) alkyle étant tel que défini ci-dessus; plus particulièrement, les termes "carbalcoxy", "carbométhoxy" et "carbéthoxy" sont utilisés ici comme synonymes des termes alkoxycarbonyle, méthoxycarbonyle et éthoxycarbonyle.

Le terme "($C_1$-$C_6$) alcanoyle" désigne le groupe carbonyle substitué par un ($C_1$-$C_6$) alkyle tel que défini ci-dessus.

Le terme "halogène" désigne les quatre halogènes courants, à savoir le fluor, le chlore, le brome et l'iode, le fluor et, plus particulièrement, le chlore étant préférés.

Les composés de formule I ci-dessus peuvent être sous une forme optiquement inactive ou sous une forme optiquement active choisie parmi celles des énantiomères, des diastéréoisomères et de leurs mélanges. Tous ces composés et leurs sels pharmaceutiquement acceptables peuvent être utilisés dans le cadre de la présente invention.

Les sels pharmaceutiquement acceptables des composés de formule I, par exemple le chlorhydrate, le bromhydrate, le sulfate, l'hydrogénosulfate, le dihydrogénophosphate, le méthanesulfonate, le méthylsulfate, l'oxalate, le maléate, le fumarate, le naphtalène-2-sulfonate, sont ceux qui sont obtenus par traitement de la base libre, dissoute par exemple dans un alcool tel que l'isopropanol, avec une solution de l'acide choisi dans le même solvant.

Lorsque le composé de formule I possède un groupe carboxyle libre, son caractère amphotère permet la préparation de sels soit avec des acides soit avec des bases pharmaceutiquement acceptables. Les sels avec des bases pharmaceutiquement acceptables sont de préférence ceux avec des métaux alcalins tels que le sodium, mais les sels avec des bases organiques, tels que le sel avec le trométamol, sont également convenables.

Sont préférés les composés de formule I ci-dessus, dans laquelle

- n est 1 ou 2;
- A est un groupe phényle, 2-fluorophényle, 3-chlorophényle ou 3-trifluorométhylphényle;
- R' est
  . un groupe fonctionnel choisi parmi les groupes hydroxy, carbalcoxy inférieur, carboxy, carbamoyle; ou
  . un groupe O-R, R étant un groupe ($C_1$-$C_6$) alkyle ou ($C_2$-$C_6$) alcényle substitué par un groupe fonctionnel choisi parmi les groupes carbalcoxy dont le groupe alkyle est en $C_1$-$C_6$ et carboxy;
- R'' et X sont l'hydrogène;
- W est une liaison directe;
- Y est l'hydrogène ou un groupe A'-CH(OH)-$CH_2$-, où A' est un groupe phényle, 2-fluorophényle, 3-chlorophényle ou 3-trifluorométhylphényle identique à A;
- Z est un alkyle inférieur, notamment méthyle.

Plus particulièrement, des principes actifs avantageux pour l'utilisation selon la présente invention sont des analogues de phényléthanolamines représentés par la formule I ci-dessus, dans laquelle

- n est 1 ou 2;
- A est un groupe phényle, 3-chlorophényle ou 3-trifluorométhylphényle;
- R' est un groupe hydroxy, carbométhoxy, carbéthoxy, carboxy, carbamoyle, carboxyméthoxy, carbométhoxyméthoxy ou carbéthoxyméthoxy;
- R'' et X sont l'hydrogène;
- W est une liaison directe;
- Y est l'hydrogène ou un groupe A'-CH(OH)-$CH_2$- où A', identique à A, est phényle; et
- Z est méthyle. Parmi ces composés, on peut citer les composés suivants : N-[2-(4-carbométhoxyphényl)-1-méthyléthyl]-2-hydroxy-2-phényléthylamine ou une N-[2-(4-carboxyphényl)-1-méthyléthyl]-2-hydroxy-2-phényléthylamine, et leurs sels pharmaceutiquement acceptables. Des principes actifs particulièrement avantageux sont
- la N-[2-(4-carbométhoxyphényl)-1-méthyléthyl]-2-hydroxy-2-phényléthylamine, ci-après désignée <u>"COMPOSE A"</u>, décrite dans l'Exemple 21 du brevet européen 6735, sous forme de mélange de diastéréoisomères de plus bas point de fusion préparé comme illustré dans la "Description 15" du brevet européen 21636; et ses sels pharmaceutiquement acceptables, notamment le fumarate neutre;
- la N-[2-(4-carboxyphényl)-1-méthyléthyl]-2-hydroxy-2-phényléthylamine, ci-après désignée <u>"COMPOSE B"</u>, préparée par saponification du Composé A, et ses sels pharmaceutiquement acceptables;
- la (R,S)-N-(2-phényl-2-hydroxyéthyl)-1-méthyl-3-(4-hydroxyphényl)propylamine, ci-après désignée <u>"COMPOSE C"</u>, décrite dans l'Exemple 10 du brevet USA 4,391,826; et ses sels pharmaceutiquement acceptables, notamment le chlorhydrate;

- la (R,S)-N-(2-phényl-2-hydroxyéthyl)-1-méthyl-3-(4-aminocarbonylphényl)propylamine, ci-après désignée "COMPOSE D", décrite dans l'Exemple 15 du brevet USA 4,391,826; et ses sels pharmaceutiquement acceptables, notamment le chlorhydrate;
- le p-[(R)-3-[bis-[(R)-béta-hydroxyphénéthyl]amino]butyl]benzamide, ci-après désigné "COMPOSE E", décrit dans l'Exemple 12 de la demande de brevet européen 101069, et ses sels pharmaceutiquement acceptables, notamment le maléate et le fumarate;
- le p-[(S)-3-[bis-[(R)-béta-hydroxyphénéthyl]amino]butyl]benzamide, ci-après désigné "COMPOSE F", décrit dans l'Exemple 8 de la demande de brevet européen 101069; et ses sels pharmaceutiquement acceptables;
- la (RR,SS)-N-[2-(4-carbométhoxyphényl)-1-méthyléthyl]-2-hydroxy-2-(3-trifluorométhylphényl)-éthanamine, ci-après désignée "COMPOSE G", décrite dans l'Exemple 19 du brevet européen 40915, et ses sels pharmaceutiquement acceptables, notamment le chlorhydrate;
- la (RR,SS)-N-[2-(4-carboxyphényl)-1-méthyléthyl]-2-hydroxy-2-(3-trifluorométhylphényl)éthanamine, ci-après désignée "COMPOSE H", préparée par saponification du Composé G; et ses sels pharmaceutiquement acceptables;
- la N-[2-(4-carbométhoxyméthoxyphényl)-1-méthyléthyl]-2-hydroxy-2-(3-chlorophényl)éthanamine, ci-après désignée "COMPOSE J", décrite dans l'Exemple 6 du européen 23385, et ses sels pharmaceutiquement acceptables, notamment le bromhydrate de sa forme (RR,SS) ("Description 21" du brevet européen 70133);
- la N-[2-(4-carboxyméthoxyphényl)-1-méthyléthyl]-2-hydroxy-2-(3-chlorophényl)éthanamine, ci-après désignée "COMPOSE K", préparée par saponification du Composé J; et ses sels pharmaceutiquement acceptables.

Les composés de formule I ci-dessus peuvent être préparés comme décrit dans les demandes de brevet européen publiées sous les numéros 6735, 7204, 21636, 23385, 25331, 28105, 40000, 40915, 52963, 61907, 63004, 66351, 68669, 70133, 70134, 89154, 91749, 95827, 99707, 101069, 140359, 146392, 164700, 170121, 170135, 171519, dans le brevet belge 900.983, dans la demande de brevet anglais 2133986, dans la demande de brevet australien 84/31944, dans la demande de brevet internationale WO 84/00956 et dans le brevet USA 4,391,826.

## ACTIVITE BIOLOGIQUE

### COLON ISOLE DE RAT

On sacrifie des rats mâles, non à jeun, pesant de 250 à 300 g. La partie proximale du côlon, consistant en un segment de 2 à 3 cm environ, est prélevée et suspendue dans une cuve à organes de 20 ml contenant une solution de Krebs-Ringer ayant la composition mM suivante: NaCl 118,4; KCl 4,7; $CaCl_2$ 2,45; $MgSO_4$ 1,16; $NaH_2PO_4$ 3,7; Glucose 5,6; $NaHCO_3$ 30,9. La solution est aérée par un mélange d'oxygène (95%) et de $CO_2$ (5%) et sa température maintenue constante à 37°C. Les segments de côlon, soumis à une traction de 1 g environ, se contractent spontanément. Les composés à l'étude sont ajoutés après stabilisation de la préparation (2h). On détermine la concentration minimale inhibant totalement les contractions rythmiques de la préparation ($CI_{100}$).

Dans le Tableau I est indiquée la $CI_{100}$ molaire de trois principes actifs représentatifs de la présente invention inhibant la motilité spontanée du côlon de rat.

TABLEAU I

| Composé testé | $CI_{100}$ /M/ |
|---|---|
| Composé A | $2,5 . 10^{7}$ |
| Composé B | $2,5 . 10^{6}$ |
| Composé C | $1 . 10^{6}$ |

MOTRICITE INTESTINALE CHEZ LE RAT ANESTHESIE

On anesthésie des rats mâles CD (Charles River, Italie) pesant 300-350 g, à jeun depuis 18 heures, avec de l'uréthane d'éthyle (1,2 g/kg/5 ml, i.p.) et on maintient leur température corporelle (37 °C ± 0,5) constante pendant toute la durée de l'expérience. La motricité est enregistrée avec des micro-capteurs à jauges de contraintes qui sont fixés sur la partie proximale du côlon à 2-3 cm de la jonction iléo-caecale. Les micro-capteurs (0,8 cm x 0,5 cm) sont préparés selon la méthode de X.B. Pascaud et al. (Am. J. Physiol. 1978, 235, E532-E538). Les composés essayés sont administrés par voie intraveineuse (veine fémorale) (1 ml/kg) ou par voie intraduodénale (2 ml/kg) lorsque le tracé de la motilité de base est régulier pendant au moins 30 minutes. Le signal électrique correspondant à l'activité mécanique du côlon est filtré (5 Hz) et enregistré sur un polygraphe muni d'un ordinateur pour l'analyse successive des données. Les paramètres considérés sont le tonus moyen et l'énergie associée à la contraction (signal électrique intégré).

Dans le Tableau II sont indiquées les doses efficaces minimales (DEM) extrapolées à partir de la droite log dose en fonction de la réponse évaluée sur l'énergie associée à la contraction pendant la période de 10 minutes après l'administration des composés.

TABLEAU II

| Principe actif | DEM (mg/kg) | |
|---|---|---|
| | i.v. | i.d. |
| Composé A | 0,15 | 0,3 |
| Composé B | 0,07 | 0,4 |
| Composé C | 0,1 | 3,5 |

MOTRICITE GASTRO-INTESTINALE ET COLIQUE CHEZ LE CHIEN

Cette étude est réalisée chez des chiens adultes pesant 9 à 15 kg. Les animaux sont équipés de jauges de contrainte fixées sur le côlon à 2, 8 et 12 cm de la valvule iléo-caeco-colique. Des groupes d'électrodes sont implantés sur l'estomac et l'intestin grêle :

- Antre (pylore - 5 cm)
- Bulbe duodénal (pylore + 5 cm) et/ou duodénum (pylore + 12 cm)
- Jéjunum
- Iléon

Une semaine après l'intervention chirurgicale, les enregistrements de l'électromyogramme gastrique et intestinal et du mécanogramme colique sont réalisés en continu. Un enregistrement direct de l'activité électrique est obtenu sur enregistreur type Alvar, à grande vitesse de déroulement de papier (1,5 cm/minute). L'activité rapide est aussi dérivée sur un enregistreur potentiométrique à faible vitesse de déroulement de papier (6 cm/h) après quantification et sommation par période de 20 secondes. L'enregistrement du mécanogramme colique est réalisé sur enregistreur potentiométrique. L'indice de motricité colique correspondant à la surface déterminée par la ligne de base et la courbe des pics de contractions est calculé, pour des périodes de 20 minutes, par un système d'analyse informatique utilisant un micro-ordinateur du type Tandy TRS 80.

Les composés à tester sont administrés soit à l'animal à jeun depuis 17 h, soit 2 h ou 15 h après le repas, à savoir en début ou en fin de la réponse motrice post-prandiale. Dans le cas des expérimentations à jeun, l'administration est effectuée soit pendant la phase d'activité irrégulière des complexes myoélectriques

migrants (CMM), soit pendant la phase de repos, 10 minutes après la phase régulière d'un complexe.

(a) Profil moteur gastro-intestinal témoin.

Chez le chien à jeun depuis 17 h, l'activité électrique rapide de l'intestin est constituée de salves de potentiels se surimposant aux ondes lentes selon deux modalités: de façon aléatoire et surchargeant 30 à 70% des ondes lentes (activité irrégulière) ou surimposant systématiquement toutes les ondes lentes pendant 5-10 minutes (activité régulière).

Cette activité présente une organisation cyclique en complexe myoélectrique migrant: la phase d'activité irrégulière (50-70 minutes environ) est suivie d'une phase d'activité régulière (5-10 minutes). Une période de silence succède pendant 20-30 minutes à cette période active. Ces CMM apparaissent environ toutes les 90 à 120 minutes.

L'activité électrique de l'estomac est aussi organisée en cycles: une période active concomitante du complexe myoélectrique du duodénum (le maximum d'activité se rencontre pendant la phase d'activité régulière): une période de silence jusqu'à la phase d'activité irrégulière du complexe suivant.

L'ingestion d'un repas standard induit immédiatement et pendant 17 h environ la disparition de cette organisation cyclique tant gastrique qu'intestinale, au profit d'une activité irrégulière permanente. La réorganisation en complexe myoélectrique débute par les portions distales de l'intestin grêle.

(b) Profil moteur colique.

Le mécanogramme colique est caractérisé par des contractions puissantes et de grande amplitude correspondant, sur le plan électrique, à des salves de potentiels d'une durée de 7 à 10 secondes (LSB, de l'anglais "Long Spike Burst"). Ces contractions sont regroupées en courtes périodes apparaissant toutes les 20 à 40 minutes. Leur fréquence est plus élevée sur le côlon proximal (2,8 ± 0,3/h) que sur le côlon distal (2,1 ± 0,4/h).

L'ingestion d'un repas induit sur l'ensemble du côlon une contraction additionelle correspondant au réflexe gastro-colique avec augmentation de l'index de motricité sur le côlon proximal. Une période d'hypomotricité d'environ 60-70 minutes fait généralement suite à cette réponse immédiate. Puis, une augmentation de l'activité colique est observée pendant 10-12 heures avec augmentation de la fréquence des périodes de contractions du côlon. L'indice de motricité est sensiblement doublé.

(c) Effets du Composé A.

Les effets du Composé A sur l'intestin grêle, testé chez deux chiens à jeun traités avec le même composé aux doses de 0,1, 0,2, et 1 mg/kg per os, sont caractérisés par la disparition durable des complexes myoélectriques migrants, aussi bien sur le duodénum que sur le jéjunum. L'activité gastrique est peu affectée. Sur le côlon, on observe une inhibition de la motricité de plus courte durée que pour l'intestin grêle.

TOXICITE

Les composés de formule I et leurs sels pharmaceutiquement acceptables sont peu toxiques. Leur toxicité aigue est bien inférieure à la dose prévue pour le traitement thérapeutique.

En particulier, certains composés de formule I ont été indiqués comme agents thérapeutiques potentiels dans le traitement de l'obésité (Nature, 1984, 309, 163-165) et un d'entre eux a été essayé en clinique humaine (Int. J. Obesity, 1985, 9, 230; ibid. 1985, 9, 231).

La présente invention concerne également, selon un autre de ses aspects, une méthode pour la prophylaxie et/ou le traitement de troubles gastro-intestinaux chez les mammifères, ladite méthode étant caractérisée en ce qu'on administre auxdits mammifères, qui nécessitent de ladite prophylaxie et/ou traitement, une quantité prophylactique et/ou efficace d'un composé de formule I ou d'un de ses sels pharmaceutiquement acceptables.

Les troubles qui peuvent être traités par les composés de formule I ci-dessus comprennent notamment ceux qui sont dûs à des contractions du muscle lisse, plus particulièrement le côlon irritable et les spasmes qui accompagnent l'ulcère peptique.

Les composés de formule I ci-dessus et leur sels pharmaceutiquement acceptables sont utilisés, selon la présente invention, à des doses journalières de 0,01 à 10 mg par kilo de poids corporel du mammifère à traiter, de préférence à des doses journalières de 0,1 à 5 mg/kg. Chez l'être humain, la dose peut varier de préférence de 0,5 mg à 500 mg par jour, plus particulièrement de 2,5 à 250 mg selon l'age du sujet à traiter, du type de traitement, prophylactique ou curatif, et de la gravité de l'affection ou du trouble gastro-intestinal. Les composés de formule I sont généralement administrés en unités de dosage de 0,1 à 100 mg, de préférence de 0,5 à 50 mg de principe actif, une à cinq fois par jour.

Lesdites unités de dosage sont de préférence formulées dans des compositions pharmaceutiques dans lesquelles le principe actif est mélangé avec un excipient pharmaceutique.

Ainsi, selon un autre de ses aspects, la présente invention concerne des compositions pharmaceutiques pour le traitement de troubles gastro-intestinaux associés à une contraction du muscle lisse, renfermant, en tant que principe actif, un composé de formule I ci-dessus ou un de ses sels pharmaceutiquement acceptables.

Dans les compositions pharmaceutiques de la présente invention pour l'administration orale, sublinguale, sous-cutanée, intramusculaire, intraveineuse, transdermique ou rectale, les ingrédients actifs de formule I ci-dessus ou leurs sels peuvent être administrés sous formes unitaires d'administration, en mélange avec des supports pharmaceutiques classiques, aux animaux et aux êtres humains. Les formes unitaires d'administration appropriées comprennent les formes par voie orale,telles que les comprimés, les gélules, les poudres, les granules et les solutions ou suspensions orales, les formes d'administration sublinguale et buccale, les formes d'administration sous-cutanée, intramusculaire ou intraveineuse et les formes d'administration rectale.

Les compositions de la présente invention contiennent l'unité de principe actif illustré ci-dessus, en mélange avec l'excipient pharmaceutique.

La composition ainsi obtenue peut être administrée 1 à 5 fois par jour comme indiqué ci-dessus.

Lorsqu'on prépare une composition solide sous forme de comprimés, on mélange le principe actif avec un véhicule pharmaceutique tel que la gélatine, l'amidon, le lactose, le stéarate de magnésium, le talc, la gomme arabique ou analogues. On peut enrober les comprimés de saccharose ou d'autres matières appropriées ou encore on peut les traiter de telle sorte qu'ils aient une activité prolongée ou retardée et qu'ils libèrent d'une façon continue une quantité prédéterminée de principe actif.

On obtient une préparation en gélules en mélangeant le principe actif avec un diluant et un lubrifiant et en versant le mélange obtenu dans des gélules molles ou dures.

Une préparation liquide sous forme de sirop ou d'elixir ou pour l'administration en gouttes peut contenir le principe actif conjointement avec un édulcorant, acalorique de préférence, du méthylparaben et du propylparaben comme antiseptiques, ainsi qu'un agent donnant du goût et un colorant approprié.

Les poudres ou les granules dispersibles dans l'eau peuvent contenir l'ingrédient actif en mélange avec des agents de dispersion ou des agents mouillants, ou des agents de mise en suspension, comme la polyvinylpyrrolidone, de même qu'avec des édulcorants ou des correcteurs du goût.

Pour une administration rectale, on recourt à des suppositoires qui sont préparés avec des liants fondant à la température rectale, par exemple du beurre de cacao ou des polyéthylèneglycols.

Pour une administration parentérale, on utilise des suspensions aqueuses, des solutions salines isotoniques ou des solutions stériles et injectables qui contiennent des agents de dispersion et/ou des mouillants pharmacologiquement compatibles, par exemple le propylèneglycol ou le butylèneglycol.

Le principe actif peut être formulé également sous forme de microcapsules ou de microémulsions, éventuellement avec un ou plusieurs supports ou additifs.

Les compositions de la présente invention peuvent contenir, à côté des produits de formule I ci-dessus ou d'un de leurs sels pharmaceutiquement acceptables, d'autres principes actifs tels que, par exemple, des analgésiques, des tranquillisants ou d'autres médicaments qui peuvent être utiles dans le traitement des troubles gastro-intestinaux.

Les exemples suivants illustrent l'invention sans toutefois la limiter.

PREPARATION 1 - Composé A.

(a) On chauffe au reflux pendant une heure un mélange de 9 g de 2-amino-1-phényléthanol et 12,7 g de 4-carbométhoxyphénylacétone dans 150 ml d'éthanol absolu, puis on refroidit et on ajoute 1 g de palladium sur charbon. On hydrogène à 4,053 bar à 60 °C pendant 5 heures. On filtre, on évapore à sec, on reprend le résidu avec 40 ml de méthanol et 40 ml d'éther éthylique et on laisse la solution à 0-4 °C pendant 48 heures. Après cristallisation et filtration, on obtient 5,5 g de N-[2-(4-carbométhoxyphényl)-1-méthyléthyl]-2-hydroxy-2-phényléthylamine; p.f. 120-125 °C (par recristallisation dans le méthanol on obtient 4,4 g de produit pur, mélange d'isomères à plus haut point de fusion; p.f. 126-128 °C).

(b) On évapore à sec les eaux mères de cristallisation provenant de l'étape (a). On obtient 13 g d'huile brute qui est dissoute dans 15 ml d'éther éthylique et 30 ml de n-hexane. Le solide cristallin qui se forme (9 g) fond à 81-84 °C. On dissout ce solide dans 30 ml d'éthanol chaud, on ajoute 100 ml d'eau chaude à 80 °C et l'on obtient ainsi une huile qui cristallise et est séparée par filtration. On obtient ainsi 7,2 g de N-[2-(4-carbométhoxyphényl)-1-méthyléthyl]-2-hydroxy-2-phényléthylamine, mélange d'isomères de plus bas point de fusion; p.f. 84-86 °C (Composé A).

En ajoutant à 0,02 moles de la base ainsi obtenue 0,01 mole d'acide fumarique dissous dans l'eau, on obtient le fumarate neutre du Composé A.

PREPARATION 2 - Composé B.

On chauffe au reflux pendant une heure un mélange de 9,2 g d'hydroxyde de sodium, 60 ml d'eau, 60 ml d'ethanol et 7,2 g de Composé A, puis on évapore l'éthanol, on filtre la phase aqueuse et on l'acidifie à pH 5 avec de l'acide chlorhydrique. On sépare par décantation le gel obtenu, on le dissout dans l'éthanol et on évapore le solvant à sec. On reprend le résidu avec de l'éthanol chaud, on filtre, on ajoute de l'isopropanol. On obtient ainsi 1,5 g de N-[2-(4-carboxyphényl)-1-méthyléthyl]-2-hydroxy-2-phényléthylamine, mélange d'isomères de plus bas point de fusion; p.f. 170-172 ° C (Composé B).

En opérant comme décrit ci-dessus, à partir de l'isomère de plus haut point de fusion obtenu dans la PREPARATION 1 (a), on obtient la N-[2-(4-carboxyphényl)-1-méthyléthyl]-2-hydroxy-2-phényléthylamine, mélange d'isomères de plus haut point de fusion, qui cristallise du méthanol; p.f. 205-207 ° C.

PREPARATION 3 - Composé H.

A partir du Composé G, selon le mode opératoire décrit dans la PREPARATION 2, on obtient la (RR,SS)-N-[2-(4-carboxyphényl)-1-méthyléthyl]-2-hydroxy-2-(3-trifluorométhylphényl)éthanamine (Composé H).

PREPARATION 4 - Composé K.

A partir du Composé J, selon le mode opératoire décrit dans la PREPARATION 2, on obtient la N-[2-(4-carboxyméthoxyphényl)-1-méthyléthyl]-2-hydroxy-2-(3-chlorophényl)éthanamine (Composé K).

EXEMPLE 1.

On prépare des gélules à base d'un des principes actifs de formule I, ayant la composition suivante:

| | |
|---|---|
| Composé A (comme fumarate neutre) | 17,5 mg |
| lactose | 120,0 mg |
| stéarate de magnésium | 5,0 mg |

en mélangeant intimement les ingrédients ci-dessus et en versant le mélange dans des gélules de gélatine dure. Ces gélules contiennent 15 mg de principe actif base libre.

EXEMPLE 2.

On prépare des comprimés à base d'un des principes actifs de formule I, ayant la composition suivante:

| | |
|---|---|
| Composé B | 20 mg |
| lactose | 100 mg |
| cellulose microcristalline | 30 mg |
| amidon de mais séché | 40 mg |
| stéarate de magnesium | 5 mg |

en broyant le principe actif jusqu'à une dimension particulaire de 0,4 mm, en le faisant passer à travers un tamis de 0,4 mm d'ouverture de maille, en mélangeant la matière broyée avec les autres constituants et en comprimant pour former les comprimés.
De la même façon, on prépare des comprimés contenant 40 mg de principe actif.

EXEMPLE 3.
En opérant comme décrit dans l'Exemple 2 ci-dessus, on prépare des comprimés à base d'un des principes actifs de formule I ayant la composition suivante:

| | |
|---|---|
| Composé A (comme fumarate neutre) | 58,3 mg |
| lactose | 95,0 mg |
| amidon de mais séché | 100,0 mg |
| talc | 4,5 mg |
| stéarate de magnesium | 0,5 mg |

Ces comprimés contiennent 50 mg de principe actif base libre.

EXEMPLE 4.

10.000 gélules avec un teneur en principe actif base libre de 50 mg sont préparées à partir des constituants suivants: 583 g de fumarate neutre du Composé A, 495 g de cellulose microcristalline, 5 g de gel de silice amorphe. Les constituants ci-dessus sont bien mélangés et introduits dans des gélules de gélatine dure de dimension 4.

EXEMPLE 5.

On prépare une solution aqueuse stérile appropriée pour une utilisation parentérale en ampoules à base d'un des principes actifs de formule I, ayant la composition suivante:

| | |
|---|---|
| Composé G (comme chlorhydrate) | 32,8 mg |
| chlorure de sodium | 5,0 mg |
| eau distillée q.s.p. | 2,0 ml |

Chaque ampoule contient 30 mg de principe actif base libre.

EXEMPLE 6.

On prépare des suppositoires à base d'un des principes actifs de formule I, ayant la composition suivante:

| | |
|---|---|
| Composé G | 50 mg |
| lactose | 250 mg |
| Witepsol® W 45 q.s.p. | 1,7 g |

On mélange la substance active avec le lactose et on met le mélange uniformément en suspension dans la masse pour suppositoires fondue. On verse la suspension dans des moules refroidis pour former des suppositoires d'un poids de 1,7 g.

EXEMPLE 7.

On prépare des comprimés à base d'un des principes actifs de formule I, ayant la composition suivante:

| | |
|---|---|
| Composé J (commme bromhydrate) | 30,8 mg |
| lactose | 95,0 mg |
| amidon de mais séché | 45,0 mg |
| silice colloidale | 2,0 mg |
| amidon soluble | 5,0 mg |
| stéarate de magnésium | 3,0 mg |

On mélange la substance active avec une partie des adjuvants et on granule le mélange avec une solution d'amidon soluble dans l'eau. Après séchage du granulat, on ajoute le reste des adjuvants et on forme des tablettes par compression. Les comprimés ainsi obtenus contiennent 25 mg de principe actif base libre.

## Revendications

1. Utilisation d'un analogue de phényléthanolamine ou d'un de ses sels pharmaceutiquement acceptables pour la préparation de compositions pharmaceutiques destinées à la prophylaxie et/ou au traitement des troubles gastro-intestinaux associés à une contraction du muscle lisse, ledit analogue de phényléthanolamine ayant la formule

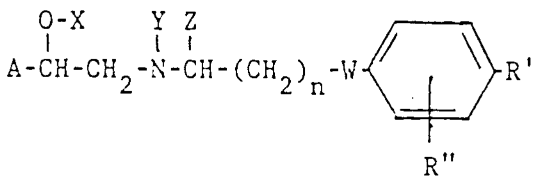

I

dans laquelle:

- n est 1,2 ou 3;
- A représente un groupe benzofuran-2-yle ou un groupe phényle non substitué ou substitué par un ou deux atomes d'halogène ou par un groupe $(C_1-C_6)$ alkyle ou trifluorométhyle;
- R' représente
  . l'hydrogène;
  . un groupe $(C_1-C_6)$ alkyle;
  . un groupe fonctionnel choisi parmi les groupes suivants: hydroxy; $(C_1-C_6)$ alcoxy; $(C_2-C_6)$ alcenyloxy; $(C_2-C_6)$ alcynyloxy; $(C_3-C_8)$ cycloalkyloxy; $(C_3-C_8)$ cycloalkyl-$(C_1-C_6)$ alcoxy; benzyloxy; phénoxy; mercapto; [$(C_1-C_6)$ alkyle]thio; [$(C_2-C_6)$ alcenyle]thio; [$(C_2-C_6)$ alcynyle]thio; $(C_3-C_8)$ cycloalkylthio; [$(C_3-C_8)$ cycloalkyl-$(C_1-C_6)$ alkyle]thio; benzylthio; phénylthio; [$(C_1-C_6)$ alkyle]sulfinyle; [$(C_2-C_6)$ alcényle]sulfinyle; [$(C_2-C_6)$ alcynyle]sulfinyle; $(C_3-C_8)$ cycloalkylsulfinyle; [$(C_3-C_8)$ cycloalkyl-$(C_1-C_6)$ alkyle]sulfinyle; benzylsulfinyle; phénylsulfinyle; [$(C_1-C_6)$ alkyle]sulfonyle; [$(C_2-C_6)$ alcenyle]sulfonyle; [$(C_2-C_6)$ alcynyle]sulfonyle; $(C_3-C_8)$ cycloalkylsulfonyle; [$(C_3-C_8)$ cycloalkyl-$(C_1-C_6)$ alkyle]sulfonyle; benzylsulfonyle; phénylsulfonyle; cyano; nitro; amino non substitué ou substitué par un ou deux radicaux, identiques ou différents, choisis parmi les groupes $(C_1-C_6)$ alkyle, $(C_2-C_6)$ alcenyle, $(C_2-C_6)$ alcynyle, $(C_3-C_8)$ cycloalkyle, $(C_3-C_8)$ cycloalkyl-$(C_1-C_6)$ alkyle, benzyle, phényle; carboxy; carbalcoxy dont le groupe alkyle est en $C_1-C_6$; [$(C_2-C_6)$ alcenyloxy]carbonyle; [$(C_2-C_6)$ alcynyloxy]carbonyle; $(C_3-C_8)$ cycloalkyloxy-carbonyle; [$(C_3-C_8)$ cycloalkyl-$(C_1-C_6)$ alcoxy]carbonyle; benzyloxycarbonyle; phénoxycarbonyle; carbamoyle non substitué ou substitué sur le groupe amino par un ou deux radicaux, identiques ou différents, choisis parmi les groupes $(C_1-C_6)$ alkyle, $(C_2-C_6)$ alcenyle, $(C_2-C_6)$ alcynyle, $(C_3-C_8)$ cycloalkyle, $(C_3-C_8)$ cycloalkyl-$(C_1-C_6)$ alkyle, benzyle, phényle;
  . un radical R choisi parmi les groupes $(C_1-C_6)$ alkyle substitués par un groupe fonctionnel, les groupes $(C_2-C_6)$ alcenyle substitués par un groupe fonctionnel, les groupes $(C_2-C_6)$ alcynyle substitués par un groupe fonctionnel, les groupes phényl [$(C_1-C_6)$ alkyle] substitués sur le groupe phényle par un $(C_1-C_6)$ alkyle ou par un groupe fonctionnel, les groupes phényl [$(C_2-C_6)$ alkenyle] substitués sur le groupe phényle par un $(C_1-C_6)$ alkyle ou par un groupe fonctionnel, les groupes phényl [$(C_2-C_6)$ alkynyle] substitués sur le groupe phényle par un $(C_1-C_6)$ alkyle ou par un groupe fonctionnel, les groupes benzyle substitués sur le groupe phényle par un $(C_1-C_6)$ alkyle ou par un groupe fonctionnel, ou les groupes phényle non substitués ou substitués par un $(C_1-C_6)$ alkyle ou par un groupe fonctionnel, le groupe fonctionnel étant comme définis ci-dessus;
  . un groupe O-R, S-R, SO-R ou $SO_2$-R, R étant tel que défini ci-dessus;
  . un groupe NRR°, où R est tel que défini ci-dessus et R° représente l'hydrogène ou est tel que défini ci-dessus pour R, ou bien R et R° forment, avec l'azote auquel ils sont liés, un groupe choisi parmi les groupes pyrrolidino, pipéridino et morpholino;
  . un groupe COOR ou CO-SR, R étant tel que défini ci-dessus;
  . un groupe CONRR°, où R est tel que défini ci-dessus et R° représente l'hydrogène ou a la même signification que R, ou bien R et R° forment, avec l'azote auquel ils sont liés, un groupe choisi parmi les groupes pyrrolidino, pipéridino et morpholino;
  . un groupe $SO_2$NRR°, où R est tel que défini ci-dessus; et R° représente l'hydrogène ou a la même signification que R, ou bien R et R° forment, avec l'azote auquel ils sont liés, un groupe choisi parmi les groupes pyrrolidino, pipéridino et morpholino;

- R'' représente
  . l'hydrogène;
  . un halogène;
  . un ($C_1$-$C_6$) alkyle
  . un groupe fonctionnel tel que défini ci-dessus;
  . un groupe O-R, R étant tel que défini ci-dessus;
  . un groupe COOR, R étant tel que défini ci-dessus;
  . un groupe CONRR°, où R est tel que défini ci-dessus et R° représente l'hydrogène ou a la même signification que R, ou bien R et R° forment, avec l'azote auquel ils sont liés, un groupe choisi parmi les groupes pyrrolidino, pipéridino et morpholino;
- W représente une liaison directe ou un atome d'oxygène;
- X représente l'hydrogène, un ($C_1$-$C_6$) alkyle ou un ($C_1$-$C_6$) alkylcarbonyle;
- Y représente l'hydrogène ou un groupe A'-CH(OH)-$CH_2$-, A' étant identique à A, mais autre que benzofuran-2-yle; ou bien
- X et Y, pris ensemble, forment un groupe méthylène, éventuellement substitué par un groupe carbalcoxy dont le groupe alkyle est en $C_1$-$C_6$,; un groupe éthylène, éventuellement substitué par un groupe oxo; ou un groupe 1,3-propylène; et
- Z représente l'hydrogène ou un $C_1$-$C_6$ alkyle.

**2.** Utilisation selon la revendication 1, caractérisée en ce que l'analogue de phényléthanolamine a la formule I indiquée dans la revendication 1 dans laquelle
- n est 1 ou 2;
- A est un groupe phényle, 2-fluorophényle, 3-chlorophényle ou 3-trifluorométhylphényle;
- R' est
  . un groupe fonctionnel choisi parmi les groupes hydroxy, carbalcoxy dont le groupe alkyle est en $C_1$-$C_6$, carboxy, carbamoyle; ou
  . un groupe O-R, R étant un groupe ($C_1$-$C_6$) alkyle ou ($C_2$-$C_6$) alcényle substitué par un groupe fonctionnel choisi parmi les groupes carbalcoxy dont le groupe alkyle est en $C_1$-$C_6$, et carboxy;
- R'' et X sont l'hydrogène;
- W est une liaison directe;
- Y est l'hydrogène ou un groupe A'-CH(OH)-$CH_2$-, où A' est un groupe phényle, 2-fluorophényle, 3-chlorophényle ou 3-trifluorométhylphényle identique à A; et
- Z est un ($C_1$-$C_6$) alkyle.

**3.** Utilisation selon la revendication 1, caractérisée en ce que l'analogue de phényléthanolamine a la formule I indiquée dans la revendication 1, dans laquelle
- n est 1 ou 2;
- A est un groupe phényle, 3-chlorophényle ou 3-trifluorométhylphényle;
- R' est un groupe hydroxy, carbométhoxy, carbéthoxy, carboxy, carbamoyle, carboxyméthoxy, carbométhoxyméthoxy ou carbéthoxyméthoxy;
- R'' et X sont l'hydrogène;
- W est une liaison directe;
- Y est l'hydrogène ou un groupe A'-CH(OH)-$CH_2$- où A', identique à A, est phényle; et
- Z est méthyle.

**4.** Utilisation selon l'une quelconque des revendications 1 à 3, caractérisée en ce que l'analogue de phényléthanolamine est choisi parmi les composés ci-après :
N-[2-(4-carbométhoxyphényl)-1-méthyléthyl]-2-hydroxy-2-phényléthylamine;
N-[2-(4-carboxyphényl)-1-méthyléthyl]-2-hydroxy-2-phényléthylamine;
(R,S)-N-(2-phényl-2-hydroxyéthyl)-1-méthyl-3-(4-hydroxyphényl)-propylamine;
(R,S)-N-(2-phényl-2-hydroxyéthyl)-1-méthyl-3-(4-aminocarbonylphényl)propylamine;
p-[(R)-3-[bis-[(R)-bêta-hydroxyphénéthyl]-amino]butyl]benzamide; (RR,SS)-N-[2-(4-carbométhoxyphényl)-1-méthyléthyl]-2-hydroxy-2-(3-trifluorométhylphényl)éthanamine;
(RR,SS)-N-[2-(4-carboxyphényl)-1-méthyléthyl]-2-hydroxy-2-(3-trifluorométhylphényl)éthanamine;
N-[2-(4-carbométhoxyméthoxyphényl)-1-méthyléthyl]-2-hydroxy-2-(3-chlorophényl)éthanamine;
N-[2-(4-carboxyméthoxyphényl)-1-méthyléthyl]-2-hydroxy-2-(3-chlorophényl)éthanamine;
ou un de leurs sels pharmaceutiquement acceptables.

**5.** Utilisation selon l'une quelconque des revendications 1 à 3, caractérisée en ce que l'analogue de phényléthanolamine est le fumarate neutre de N-[2-(4-carbométhoxyphényl)-1-méthyléthyl]-2-hydroxy-2-phényléthylamine sous forme de mélange de diastéréoisomères de plus bas point de fusion.

**6.** Utilisation selon l'une quelconque des revendications 1 à 4, caractérisée en ce que l'analogue de phényléthanolamine est la (RR,SS)-N-[2-(4-carbométhoxyméthoxyphényl)-1-méthyléthyl]-2-hydroxy-2-(3-chlorophényl)éthanamine ou son bromhydrate.

**7.** Composition pharmaceutique pour la prophylaxie et/ou le traitement de troubles gastro-intestinaux associés à une contraction du muscle lisse, obtenue par utilisation d'un analogue de phényléthanolamine ou d'un de ses sels pharmaceutiquement acceptables selon l'une quelconque des revendications 1 à 6, en tant que principe actif.

**8.** Composition pharmaceutique selon la revendication 7 sous forme d'unité de dosage contenant de 0,1 à 100 mg de principe actif en mélange avec un excipient pharmaceutique.

**9.** Composition pharmaceutique selon la revendication 7 ou 8 contenant de 0,5 à 50 mg de principe actif par unité de dosage en mélange avec un excipient pharmaceutique.

## Claims

**1.** Use of a phenylethanolamine analog or a pharmaceutically acceptable salt thereof for the preparation of pharmaceutical compositions intended for the prophylaxis and/or treatment of gastrointestinal diseases associated with a smooth muscle contraction, said phenylethanolamine analog having the formula

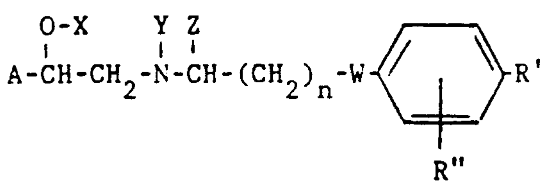

I

in which:

- n is 1, 2 or 3;
- A is a benzofuran-2-yl group or a phenyl group unsubstituted or substituted with one or two halogen atoms or with a ($C_1$-$C_6$) alkyl or a trifluoromethyl group;
- R' is
  . hydrogen;
  . a ($C_1$-$C_6$) alkyl group;
  . a functional group selected from the following groups: hydroxy; ($C_1$-$C_6$) alkoxy; ($C_2$-$C_6$) alkenyloxy; ($C_2$-$C_6$) alkynyloxy; ($C_3$-$C_8$) cycloalkyloxy; ($C_3$-$C_8$) cycloalkyl-($C_1$-$C_6$) alkoxy; benzyloxy; phenoxy; mercapto; [($C_1$-$C_6$) alkyl]thio; [($C_2$-$C_6$) alkenyl]thio; [($C_2$-$C_6$) alkynyl]thio; ($C_3$-$C_8$) cycloalkylthio; [($C_3$-$C_8$) cycloalkyl-($C_1$-$C_6$) alkyl]thio; benzylthio; phenylthio; [($C_1$-$C_6$) alkyl]-sulfinyl; [($C_2$-$C_6$) alkenyl]sulfinyl; [($C_2$-$C_6$) alkynyl]sulfinyl; ($C_3$-$C_8$) cycloalkylsulfinyl; [($C_3$-$C_8$) cycloalkyl-($C_1$-$C_6$) alkyl]sulfinyl; benzylsulfinyl; phenylsulfinyl; [($C_1$-$C_6$) alkyl]sulfonyl; [($C_2$-$C_6$) alkenyl]sulfonyl; [($C_2$-$C_6$) alkynyl]sulfonyl; ($C_3$-$C_8$) cycloalkylsulfonyl; [($C_3$-$C_8$) cycloalkyl-($C_1$-$C_6$) alkyl]sulfonyl; benzylsulfonyl; phenylsulfonyl; cyano; nitro; amino unsubstituted or substituted with one or two identical or different radicals selected from the groups consisting of ($C_1$-$C_6$) alkyl; ($C_2$-$C_6$) alkenyl; ($C_2$-$C_6$) alkynyl; ($C_3$-$C_8$) cycloalkyl; ($C_3$-$C_8$) cycloalkyl-($C_1$-$C_6$) alkyl; benzyl; phenyl; carboxy; carbalkoxy of which the alkyl group is $C_1$-$C_6$; [($C_2$-$C_6$) alkenyloxy]-carbonyl; [($C_2$-$C_6$) alkynyloxy]carbonyl; ($C_3$-$C_8$) cycloalkyloxycarbonyl; [($C_3$-$C_8$) cycloalkyl-($C_1$-$C_6$) alkoxy]carbonyl; benzyloxycarbonyl; phenoxycarbonyl; carbamoyl unsubstituted or substituted on the amino group with one or two identical or different radicals, selected from the ($C_1$-$C_6$) alkyl, ($C_2$-$C_6$) alkenyl, ($C_2$-$C_6$) alkynyl, ($C_3$-$C_8$) cycloalkyl, ($C_3$-$C_8$) cycloalkyl-($C_1$-$C_6$) alkyl, benzyl, phenyl groups;
  . a R radical selected from the ($C_1$-$C_6$) alkyl groups substituted with a functional group, the ($C_2$-$C_6$) alkenyl groups substituted with a functional group, the ($C_2$-$C_6$) alkynyl groups substituted

with a functional group, the phenyl [($C_1$-$C_6$) alkyl] groups substituted on the phenyl group with a ($C_1$-$C_6$) alkyl or a functional group, the phenyl [($C_2$-$C_6$) alkenyl] groups substituted on the phenyl group with a ($C_1$-$C_6$) alkyl or a functional group, the phenyl [($C_2$-$C_6$) alkynyl] groups substituted on the phenyl group with a ($C_1$-$C_6$) alkyl or a functional group, the benzyl groups substituted on the phenyl group with a ($C_1$-$C_6$) alkyl or a functional group, or the phenyl groups unsubstituted or substituted with a ($C_1$-$C_6$) alkyl or a functional group, the functional group being as defined hereinabove;

. a O-R, S-R, SO-R, $SO_2$-R group, R being as defined hereinabove;

. a NRR° group, where R is as defined hereinabove and R° represents hydrogen or is as defined hereinabove for R, or R and R° form, together with the nitrogen atom to which they are bound, a group selected from the pyrrolidino, piperidino and morpholino groups;

. a COOR or CO-SR group, R being as defined hereinabove;

. a CONRR° group, where R is as defined hereinabove and R° represents hydrogen or has the same meaning as R, or R and R° form, together with the nitrogen atom to which they are bound, a group selected from the pyrrolidino, piperidino and morpholino groups;

. a $SO_2$NRR° group, where R is as defined hereinabove; and R° represents hydrogen or has the same meaning as R, or R and R° form, together with the nitrogen atom to which they are bound, a group selected from the pyrrolidino, piperidino and morpholino groups;

- R'' represents
  . hydrogen;
  . a halogen;
  . a ($C_1$-$C_6$) alkyl;
  . a functional group as defined hereinabove;
  . a O-R group, R being as defined hereinabove;
  . a COOR group, R being as defined hereinabove;
  . a CONRR° group, where R is as defined hereinabove and R° represents hydrogen or has the same meaning as R, or R and R° form, together with the nitrogen atom to which they are bound, a group selected from the pyrrolidino, piperidino and morpholino groups;
- W represents a direct bond or an oxygen atom;
- X represents hydrogen, a ($C_1$-$C_6$) alkyl or a ($C_1$-$C_6$) alkylcarbonyl;
- Y represents hydrogen or a A' -CH(OH)-$CH_2$- group, A' being identical to A, but other than benzofuran-2-yl; or
- X and Y, together, form a methylene group, optionally substituted with a carbalkoxy group of which the alkyl group is $C_1$-$C_6$; an ethylene group, optionally substituted with an oxo group; or a 1,3-propylene group; and
- Z represents hydrogen or a ($C_1$-$C_6$) alkyl.

**2.** Use according to claim 1, characterized in that the phenylethanolamine analog has the formula I indicated in claim 1, in which:
- n is 1 or 2;
- A is a phenyl, 2-fluorophenyl, 3-chlorophenyl or 3-trifluoromethylphenyl group;
- R' is
  . a functional group selected from the hydroxy, carbalkoxy of which the alkyl group is $C_1$-$C_6$, carboxy, carbamoyl groups; or
  . a O-R group, R being a ($C_1$-$C_6$) alkyl group or a ($C_2$-$C_6$) alkenyl group substituted with a functional group selected from the carbalkoxy of which the alkyl group is $C_1$-$C_6$ and carboxy groups.
- R'' and X are hydrogen;
- W is a direct bond;
- Y is hydrogen or a A' -CH(OH)-$CH_2$- group, where A' is a phenyl, 2-fluorophenyl, 3-chlorophenyl or 3-trifluoromethylphenyl group, identical to A; and
- Z is a ($C_1$-$C_6$) alkyl.

**3.** Use according to claim 1, characterized in that the phenylethanolamine analog has the formula I indicated in claim 1, in which:
- n is 1 or 2;
- A is a phenyl, 3-chlorophenyl or 3-trifluoromethylphenyl group;

- R' is a hydroxy, carbomethoxy, carbethoxy, carboxy, carbamoyl, carboxymethoxy, carbomethoxymethoxy or carbethoxymethoxy group;
- R" and X are hydrogen;
- W is a direct bond;
- Y is hydrogen or a A' -CH(OH)-$CH_2$- group where A', identical to A, is phenyl; and
- Z is methyl.

**4.** Use according to any one of claims 1 to 3, characterized in that the phenylethanolamine analog is selected from the following compounds:
N-[2-(4-carbomethoxyphenyl)-1-methylethyl]-2-hydroxy-2-phenylethylamine;
N-[2-(4-carboxyphenyl)-1-methylethyl]-2-hydroxy-2-phenylethylamine;
(R,S)-N-(2-phenyl-2-hydroxyethyl)-1-methyl-3-(4-hydroxyphenyl)propylamine;
(R,S)-N-(2-phenyl-2-hydroxyethyl)-1-methyl-3-(4-aminocarbonylphenyl)propylamine;
p-[(R)-3-[bis-[(R)-beta-hydroxyphenethyl]-amino]butyl]benzamide; (RR,SS)-N-|2-(4-carbomethoxyphenyl)-1-methylethyl]-2-hydroxy-2-(3-trifluoromethylphenyl)ethanamine;
(RR,SS)-N-[2-(4-carboxyphenyl)-1-methylethyl]-2-hydroxy-2-(3-trifluoromethylphenyl)ethanamine;
N-[2-(4-carbomethoxymethoxyphenyl)-1-methylethyl]-2-hydroxy-2-(3-chlorophenyl)ethanamine;
N-[2-(4-carboxymethoxyphenyl)-1-methylethyl]-2-hydroxy-2-(3-chlorophenyl)ethenamine;
or a pharmaceutically acceptable salt thereof.

**5.** Use according to any one of claims 1 to 3, characterized in that the phenylethanolamine analog is the neutral fumarate of N-[2-(4-carbomethoxyphenyl)-1-methylethyl]-2-hydroxy-2-phenylethylamine in mixture of diastereoisomers with lower melting point.

**6.** Use according to any one of claims 1 to 4, characterized in that the phenylethanolamine analog is the (RR,SS)-N-[2-(4-carbomethoxymethoxyphenyl)-1-methylethyl]-2-hydroxy-2-(3-chlorophenyl)ethanamine or its hydrobromide.

**7.** Pharmaceutical composition for the prophylaxis and/or treatment of gastrointestinal diseases associated with a smooth muscle contraction, obtained by using a phenylethanolamine analog or a pharmaceutically acceptable salt thereof, according to any one of claims 1 to 6, as active principle.

**8.** Pharmaceutical composition according to claim 7 in dosage unit form comprising from 0.1 to 100 mg of active principle in admixture with a pharmaceutical excipient.

**9.** Pharmaceutical composition according to claim 7 or 8 comprising from 0.5 to 50 mg of active principle per unit dosage in admixture with a pharmaceutical excipient.

**Patentansprüche**

**1.** Verwendung eines Phenylethanolamin-Analogen oder eines seiner pharmazeutisch akzeptablen Salze zur Herstellung von pharmazeutischen Zusammensetzungen zur Prophylaxe und/oder Behandlung von Gastrointestinalbeschwerden, die mit einer Kontraktion des glatten Muskels verbunden sind, wobei das Phenylethanolamin-Analoge die folgende Formel besitzt:

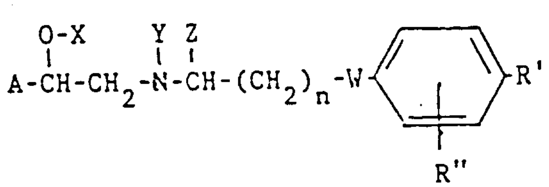

I,

worin:
- n = 1, 2 oder 3 ist;
- A eine Benzofuran-2-yl-Gruppe oder eine unsubstituierte oder mit einem oder zwei Halogenatomen oder einer $C_1$-$C_6$-Alkylgruppe oder Trifluormethylgruppe substituierte Phenylgruppe ist;
- R' bedeutet:

. Wasserstoff;
. eine $C_1$-$C_6$-Alkylgruppe;
. eine funktionelle Gruppe, die ausgewählt wird unter: Hydroxy, $C_1$-$C_6$-Alkoxy, $C_2$-$C_6$-Alkenyloxy, $C_2$-$C_6$-Alkinyloxy, $C_3$-$C_8$-Cycloalkyloxy, $C_3$-$C_8$-Cycloalkyl-$C_1$-$C_6$-Alkoxy, Benzyloxy, Phenoxy, Mercapto, [$C_1$-$C_6$-Alkylthio, [$C_2$-$C_6$-Alkenyl]thio, [$C_2$-$C_6$-Alkinyl]thio, $C_3$-$C_8$-Cycloalkylthio, [$C_3$-$C_8$-Cycloalkyl-$C_1$-$C_6$-Alky]thio, Benzylthio, Phenylthio, [$C_1$-$C_6$-Alkyl]sulfinyl, [$C_2$-$C_6$-Alkenyl]-sulfinyl, [$C_2$-$C_6$-Alkinyl]sulfinyl, $C_3$-$C_8$-Cycloalkylsulfinyl, [$C_3$-$C_8$-Cycloalkyl-$C_1$-$C_6$-Alkyl]sulfinyl, Benzylsulfinyl, Phenylsulfinyl, [$C_1$-$C_6$-Alkyl]sulfonyl, [$C_2$-$C_6$-Alkenyl]sulfonyl, [$C_2$-$C_6$-Alkinyl]-sulfonyl, $C_3$-$C_8$-Cycloalkylsulfonyl, [$C_3$-$C_8$-Cycloalkyl-$C_1$-$C_6$-Alkyl]sulfonyl, Benzylsulfonyl, Phenylsulfonyl, Cyano, Nitro, unsubstituiertes oder mit einer oder zwei gleichen oder verschiedenen Gruppen substituiertes Amino, die ausgewählt werden unter $C_1$-$C_6$-Alkyl, $C_2$-$C_6$-Alkenyl, $C_2$-$C_6$-Alkinyl, $C_3$-$C_8$-Cycloalkyl, $C_3$-$C_8$-Cycloalkyl-$C_1$-$C_6$-Alkyl, Benzyl, Phenyl, Carboxy, Carbalkoxy mit einer $C_1$-$C_6$-Alkylgruppe, [$C_2$-$C_6$-Alkenyloxy]carbonyl, [$C_2$-$C_6$-Alkinyloxy]carbonyl, $C_3$-$C_8$-Cycloalkyloxycarbonyl, [$C_3$-$C_8$-Cycloalkyl-$C_1$-$C_6$-Alkoxy]carbonyl, Benzyloxycarbonyl, Phenoxycarbonyl, unsubstituiertes oder an der Aminogruppe mit einer oder zwei gleichen oder verschiedenen Gruppen substituiertes Carbamoyl, die ausgewählt werden unter $C_1$-$C_6$-Alkyl, $C_2$-$C_6$-Alkenyl, $C_2$-$C_6$-Alkinyl, $C_3$-$C_8$-Cycloalkyl, $C_3$-$C_8$-Cycloalkyl-$C_1$-$C_6$-Alkyl, Benzyl, Phenyl;
. einen Rest R, der ausgewählt wird unter mit einer funktionellen Gruppe substituierten $C_1$-$C_6$-Alkylgruppen,mit einer funktionellen Gruppe substituierten $C_2$-$C_6$-Alkenylgruppen, mit einer funktionellen Gruppe substituierten $C_2$-$C_6$-Alkinylgruppen, Phenyl-[$C_1$-$C_6$-Alkyl]gruppen, die an der Phenylgruppe mit einer $C_1$-$C_6$-Alkylgruppe oder einer funktionellen Gruppe substituiert sind, Phenyl-[$C_2$-$C_6$-Alkenyl]gruppen, die an der Phenylgruppe mit einer $C_1$-$C_6$-Alkylgruppe oder einer funktionellen Gruppe substituiert sind, Phenyl-[$C_2$-$C_6$- Alkinyl]gruppen, die an der Phenylgruppe mit einer $C_1$-$C_6$-Alkylgroppe oder einer funktionellen Gruppe substituiert sind, Benzylgruppen, die an der Phenylgruppe mit einer $C_1$-$C_6$-Alkylgruppe oder einer funktionellen Gruppe substituiert sind, oder unsubstituierte oder mit einer $C_1$-$C_6$-Alkylgruppe oder einer funktionellen Gruppe substituierte Phenylgruppen, wobei die funktionelle Gruppe die gleiche ist wie oben definiert;
. eine O-R-, S-R-, SO-R- oder $SO_2$-R-Gruppe, wobei R das gleiche bedeutet wie oben definiert;
. eine NRR°-Gruppe, in der R das gleiche bedeutet wie oben definiert und R° Wasserstoff oder das gleiche wie oben für R definierte bedeutet, oder wobei R und R° mit dem Stickstoffatom, an das sie gebunden sind, eine Gruppe bilden, die ausgewählt wird unter Pyrrolidino, Piperidino und Morpholino;
. eine COOR- oder CO-SR-Gruppe, wobei R das gleiche bedeutet wie oben definiert;
. eine CONRR°-Gruppe, worin R das gleiche bedeutet wie oben definiert und R° Wasserstoff bedeutet oder die gleiche Bedeutung besitzt wie R, oder R und R° mit dem Stickstoffatom, an das sie gebunden sind, eine Gruppe bilden, die ausgewählt wird unter Pyrrolidino, Piperidino und Morpholino;
. eine $SO_2$NRR°-Gruppe, worin R das gleiche bedeutet wie oben definiert und R° Wasserstoff bedeutet oder die gleiche Bedeutung besitzt wie R, oder R und R° mit dem Stickstoffatom, an das sie gebunden sind, eine Gruppe bilden, die ausgewählt wird unter Pyrrolidino, Piperidino und Morpholino;

- R'' bedeutet:
. Wasserstoff;
. ein Halogen;
. eine $C_1$-$C_6$-Alkylgruppe;
. eine funktionelle Gruppe wie oben definiert;
. eine O-R-Gruppe, worin R das gleiche bedeutet wie oben definiert;
. eine COOR-Gruppe, worin R das gleiche bedeutet wie oben definiert;
. eine CONRR°-Gruppe, worin R das gleiche bedeutet wie oben definiert und R° Wasserstoff ist oder die gleiche Bedeutung besitzt wie R, oder R und R° mit dem Stickstoffatom, an das sie gebunden sind, eine Gruppe bilden, die ausgewählt wird unter Pyrrolidino, Piperidino und Morpholino;
- W eine direkte Bindung oder ein Sauerstoffatom bedeutet;
- X Wasserstoff, ein $C_1$-$C_6$-Alkyl- oder eine $C_1$-$C_6$-Alkylcarbonylgruppe bedeutet;
- Y Wasserstoff oder eine A'-CH(OH)-$CH_2$-Gruppe bedeutet, worin A' identisch mit A, aber nicht Benzofuran-2-yl ist, oder

- X und Y zusammen eine Methylengruppe bilden, die eventuell mit einer Carbalkoxygruppe mit einer $C_1$-$C_6$-Alkylgruppe,einer Ethylengruppe, die eventuell mit einer Oxogruppe substituiert ist, oder mit einer 1,3-Propylengruppe substituiert ist; und
- Z Wasserstoff oder eine $C_1$-$C_6$-Alkylgruppe bedeutet.

**2.** Verwendung nach Anspruch 1, dadurch gekennzeichnet, daß das Phenylethanolamin-Analoge die in Anspruch 1 angegebene Formel I besitzt, worin
- n = 1 oder 2;
- A eine Phenyl-, 2-Fluorphenyl-, 3-Chlorphenyl- oder 3-Trifluormethylphenyl-Gruppe ist;
- R' bedeutet:
  . eine funktionelle Gruppe, die ausgewählt wird unter Hydroxy, Carbalkoxy mit einer $C_1$-$C_6$-Alkylgruppe, Carboxy, und Carbamoyl oder
  . eine O-R-Gruppe, wobei R eine $C_1$-$C_6$-Alkylgruppe oder eine mit einer funktionellen Gruppe substituierte $C_2$-$C_6$- Alkenylgruppe ist, die ausgewählt wird unter Carbalkoxy-Gruppen mit $C_1$-$C_6$-Alkylgruppen und Carboxy;
- R'' und X Wasserstoff sind;
- W eine direkte Bindung ist;
- Y Wasserstoff oder eine A'-CH(OH)-$CH_2$-Gruppe ist, worin A' eine Phenyl-, 2-Fluorphenyl-, 3-Chlorphenyl- oder 3-Trifluormethylphenyl-Gruppe ist, die identisch ist mit A und
- Z $C_1$-$C_6$-Alkyl ist.

**3.** Verwendung nach Anspruch 1, dadurch gekennzeichnet, daß das Phenylethanolamin-Analoge die in Anspruch 1 angegebene Formel I besitzt, worin
- n = 1 oder 2;
- A eine Phenyl-, 3-Chlorphenyl- oder 3-Trifluormethylphenyl-Gruppe ist;
- R' ein Hydroxy-, Carbomethoxy-, Carbethoxy-, Carboxy-, Carbamoyl-, Carboxymethoxy-, Carbomethoxymethoxy- oder Carbethoxymethoxy-Gruppe ist;
- R'' und X Wasserstoff sind;
- W eine direkte Bindung ist;
- Y Wasserstoff oder eine A'-CH(OH)-$CH_2$-Gruppe ist, worin A', das identisch ist mit A, Phenyl ist; und
- Z eine Methyl-Gruppe ist.

**4.** Verwendung nach einem der Ansprüche 1 bis 3, dadurch gekennzeichnet, daß das Phenylethanolamin-Analoge ausgewählt wird unter den folgenden Verbindungen:
N-[2-(4-Carbomethoxyphenyl)-1-methylethyl]-2-hydroxy-2-phenylethylamin;
N-[2-(4-Carboxyphenyl)-1-methylethyl]-2-hydroxy-2-phenylethylamin;
(R,S)-N-(2-Phenyl-2-hydroxyethyl)-1-methyl-3-(4-hydroxyphenyl)-propylamin;
(R,S)-N-(2-Phenyl-2-hydroxyethyl)-1-methyl-3-(4-aminocarbonylphenyl)propylamin;
p-[(R)-3-[bis-[(R)-$\beta$-hydroxyphenetyl]-amino]butyl]benzamid;
(RR,SS)-N-[2-(4-Carbomethoxyphenyl)-1-methylethyl]-2-hydroxy-2-(3-trifluormethylphenyl)ethanamin;
(RR,SS)-N-[2-(4-Carboxyphenyl)-1-methylethyl]-2-hydroxy-2-(3-trifluormethylphenyl)ethanamin;
N-[2-(4-Carbomethoxymethoxyphenyl)-1-methylethyl]-2-hydroxy-2-(3-chlorphenyl)ethanamin;
N-[2-(4-Carboxymethoxyphenyl)-1-methylethyl]-2-hydroxy-2-(3-chlorphenyl)ethanamin,
oder eines ihrer pharmazeutisch akzeptablen Salze.

**5.** Verwendung nach einem der Ansprüche 1 bis 3, dadurch gekennzeichnet, daß das Phenylethanolamin-Analoge das neutrale Fumarat von N-[2-(4-Carbomethoxyphenyl)-1-methylethyl]-2-hydroxy-2-phenylethylamin in Form des Diastereoisomeren-Gemisches mit niedrigerem Schmelzpunkt ist.

**6.** Verwendung nach einem der Ansprüche 1 bis 4, dadurch gekennzeichnet, daß das Phenylethanolamin-Analoge (RR,SS)-N-[2-(4-Carbomethoxymethoxyphenyl)-1-methylethyl]-2-hydroxy-2-(3-chlorphenyl)-ethanamin oder sein Hydrobromid ist.

**7.** Pharmazeutische Zusammensetzung zur Prophylaxe und/oder zur Behandlung von Gastrointestinalbeschwerden, die mit einer Kontraktion des glatten Muskels einhergehen, die durch Verwendung eines Phenylethanolamin-Analogen oder eines seiner pharmazeutisch akzeptablen Salze nach einem der Ansprüche 1 bis 6 als Wirkstoff erhalten ist.

8. Pharmazeutische Zusammensetzung nach Anspruch 7 in Form der Einheitsdosis, die 0,1 bis 100 mg Wirkstoff im Gemisch mit einem Arzneimittelträger enthält.

9. Pharmazeutische Zusammensetzung nach Anspruch 7 oder 8, die 0,5 bis 50 mg Wirkstoff pro Einheitsdosis im Gemisch mit einem pharmazeutischen Träger enthält.